Europäisches Patentamt

European Patent Office

Office européen des brevets

(19)

(11) Publication number: **0 098 656**
**B1**

(12) **EUROPEAN PATENT SPECIFICATION**

(45) Date of publication of patent specification: **31.08.88**

(51) Int. Cl.⁴: **A 61 N 1/16**

(21) Application number: **83200976.5**

(22) Date of filing: **30.06.83**

(54) Earthing device.

(30) Priority: **01.07.82 NL 8202664**

(43) Date of publication of application:
**18.01.84 Bulletin 84/03**

(45) Publication of the grant of the patent:
**31.08.88 Bulletin 88/35**

(84) Designated Contracting States:
**AT BE CH DE FR GB IT LI LU NL SE**

(56) References cited:
**DE-A- 526 039**
**FR-A- 657 295**
**FR-A-1 375 858**
**FR-A-1 384 763**
**FR-A-1 417 864**
**GB-A- 322 485**
**GB-A- 650 284**
**GB-A- 664 478**

(73) Proprietor: **Gierkink, Alphons Johannes
Hendrikus
No. 35, Bernard van Meursstraat
NL-7131 XG Lichtenvoorde (NL)**

(72) Inventor: **Gierkink, Alphons Johannes
Hendrikus
No. 35, Bernard van Meursstraat
NL-7131 XG Lichtenvoorde (NL)**

(74) Representative: **Schumann, Bernard Herman
Johan et al
OCTROOIBUREAU ARNOLD & SIEDSMA
Sweelinckplein 1
NL-2517 GK The Hague (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 098 656 B1

## Description

The invention relates to an earthing device comprising an elongate electrical conductor bent in one plane in a symmetrical fashion such that its ends are spaced apart by a small distance.

Such a device is known from GB-A-322 485. The electrical conductor forming part of this prior art device is carried by an insulating support means.

It is a purpose of the invention to provide a novel device of that type. In view thereof the device according to the invention is characterized in that the middle of the conductor is connected with an earthing terminal.

For achieving optimum activity it is preferred to provide positioning means for arranging the plane of the conductor in a substantially horizontal position. Very useful is the variant in which the positioning means are constructed in the form of a ground pin fastened to the earth terminal and to be inserted into the ground in a position substantially perpendicular to the plane of the conductor.

It may be advantageous when the device embodying the invention can be carried on the body. For this purpose it may be provided with an electrically conductive carrying chain connected with the earth terminal. This carrying chain should be in contact with the skin. In this respect it is noted that even slight conductivity is sufficient in view of the extremely weak electric currents involved. From practical experience it has been found that even poorly conductive rubber soles have a conductive power which is sufficient for satisfactory operation of the device embodying the invention. It should be noted that a device embodying the invention. It should be noted that a device embodying the invention carried around the neck will occupy a more or less vertical position so that no optimum results are ensured. In view thereof it is preferred to carry the device in a substantially horizontal position on the body. Particularly suitable thereto are the shoulders and the feet.

Very satisfactory results have been obtained with a device in which the surfaces of the ends of the conductor are flat and substantially coincide with the plane of symmetry of the conductor.

In order to adapt the device to the purpose aimed at and in particular to avoid unnecessarily large dimensions it is preferred to choose the surface enclosed by the conductor on basis of the radius of the desired active zone.

It will be obvious that the strip or wire may be made of metal. Lighter and cheaper may be the variant in which the conductor is a strip or wire made of electrical insulating material coated with an electrically conductive layer.

A very important enlargement of the working zone is obtained by a variant in which the ends of the conductor are connected with the earth terminal by means of electric conductors which may be provided with an insulating sheath.

The invention will now be described with reference to the drawing of a few arbitrarily chosen embodiments. The drawing shows in:

Fig. 1 a perspective view of a first embodiment;

Fig. 2 perspective view of a second embodiment;

Fig. 3 perspective view of a third embodiment;

Fig. 4 perspective view of a fourth embodiment;

Fig. 5 perspective view of a fifth embodiment;

Fig. 6 perspective view of a sixth embodiment;

Fig. 7 perspective view of a seventh embodiment;

Fig. 8 perspective view of a eighth embodiment;

Fig. 9 perspective view of a ninth embodiment;

Fig. 10 a schematic, graphical representation of the active zone of the embodiments of figs. 1 to 7, 8 and 9.

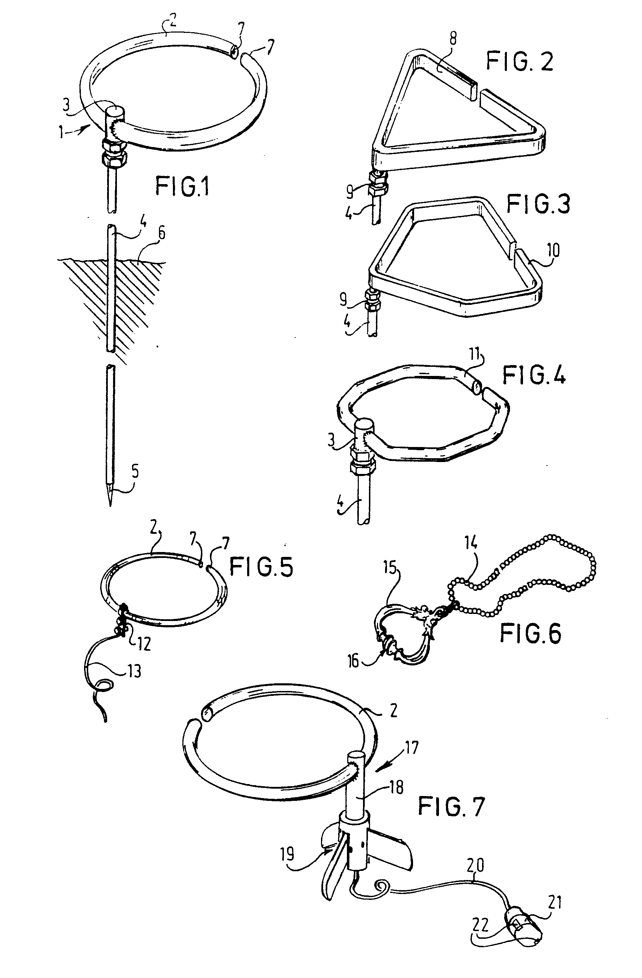

Fig. 1 shows a device 1 embodying the invention. This device 1 comprises an electrically conductive wire 2, to the middle of which is fastened an earth terminal 3. To the earth terminal 3 is secured a ground pin 4. The latter has a tip 5 at the lower end so that the pin 4 can be readily inserted into the ground in a manner such that the ground pin 4 comes into contact with humid earth or even ground water. The conductor 2 is annularly bent so that it is located in one plane, whilst its ends 7 are at a small distance from one another.

The plane of the conductor 2 is substantially normal to the direction of length of the ground pin 4 so that the conductor 2 can be readily set in a horizontal position.

Fig. 2 shows a second embodiment of the invention. In this embodiment a strip 8 has a rectangular cross-section in contrast to the first embodiment of fig. 1 in which the conductor 2 has a circular cross-section, whilst the shape of the strip is substantially a triangle with rounded-off corners. To one of the corners is fastened an earth connection 9 with the ground pin 4. On the side opposite said corner the ends of the strip 8 are slightly spaced apart.

The third embodiment shown in fig. 3 comprises a strip 10 of pentagonal shape. To one corner is fastened the earth connection 9 with the ground pin 10. On the opposite side in the middle the ends of the strip 10 are slightly spaced apart. The cross-section of the strip 10 is generally rectangular.

Fig. 4 shows a fourth embodiment. A round wire 11 has an octogonal shape. On one side the earth terminal 3 is arranged with the ground pin 4 like in the embodiment of fig. 1, whilst on the opposite side the ends of the wire 11 are located at a small distance from one another.

Fig. 5 shows a fifth, simple embodiment in which the conductor 2 is provided in the middle with an earth terminal 12 to which a ground lead 13 is conductively fastened. For practical use this simple embodiment has to be disposed in an insulated manner. Only the ground lead 13 has to be connected with the ground.

Fig. 6 shows an elegant embodiment of the device in accordance with the invention. A conductive carrying chain 14 serves to carry the conductive ring 15, which is conductively connected in the middle with the chain 14.

Symmetrically opposite said connection there is a small free space 16.

Fig. 7 shows a device 17 having an annular conductor 2 to the middle of which is secured a conductive carrying rod 18, the lower part of which is carried by a relatively heavy foot 19. To the carrying rod 18 is fastened an earthing wire 20, to the free end of which is secured an earthing plug 21. This earthing plug has the same shape as a conventional plug with earthed rim 22, but it is not provided with pins so that after insertion into a normal wall mains connector it can solely serve for earthing purposes.

All embodiments described above are constructed so that the surfaces of the ends of the strip or wire are flat and substantially coincide with the plane of symmetry of the conductive ring.

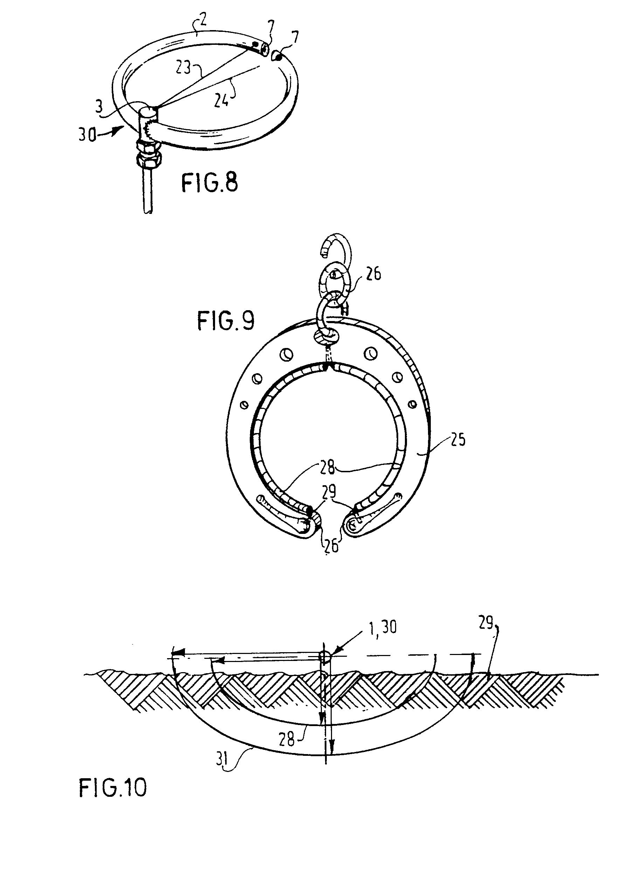

Fig. 8 shows a variant differing from the embodiment of fig. 1 by the presence of two conductors 23, 24 by which the ends 7 are conductively connected with the earth terminal 3.

Fig. 9 shows an embodiment in which a ring 25 can be carried by means of a chain 26 on the body like in the embodiment of fig. 6, whilst in accordance with the embodiment of fig. 8 the ends 26 are connected with the middle of the ring 25 by means of conductors 29 provided with an insulating sheath 28. The operation of this embodiment is fully analogous to that of fig. 8.

Fig. 10 is a schematic representation of the active zone of the devices without electric connection between the ends and the earth terminal. The curve 28 indicated the boundary of the operative range of the device of fig. 1. It is inserted into the ground 29. The device 1 is operative along a depth of, for example, 5 metres, whilst the radius of the active zone at the surface may be about 20 metres.

The curve 31 indicates the boundary of the active zone of the device 30 of fig. 8. From fig. 10, drawn approximately to scale, it appears that the activity in depth is enlarged by 100%, whilst the active radius at ground surface has increased by about 30%.

It will be obvious that the embodiments described only serve to illustrate the idea of the invention and that the implementation depends on the specific purpose of use, that is to say, whether it has to be placed in the ground, to be used indoors or to be carried on the body, whilst the dimensions may be chosen in accordance with a desired active zone.

### Claims

1. An earthing device comprising an elongate-electrical conductor (2) bent in one plane in a symmetrical fashion such that its ends (7) are spaced apart by a small distance, characterized in that the middle of the conductor (2) is connected with an earthing terminal (3).

2. A device as claimed in claim 1, characterized by positioning means for arranging the plane of the conductor in a substantially horizontal position.

3. A device as claimed in claim 2, characterized in that the positioning means are constructed in the form of a ground pin (4) fastened to the earth terminal (3) and to be inserted into the ground in a position substantially perpendicular to the plane of the conductor.

4. A device as claimed in claim 1 suitable for being carried on the body, characterized bv an electrically conductive carrying chain (14) connected with the earth terminal.

5. A device as claimed in anyone of the preceding claims, characterized in that the surfaces of the ends of the conductor are flat and substantially coincide with the plane of symmetry of the conductor.

6. A device as claimed in anyone of the preceding claims, characterized in that the surface enclosed by the conductor (2) is chosen on basis of the radius of the desired active zone.

7. A device as claimed in anyone of the preceding claims, charaacterized in that the conductor is a strip or wire made of electrical insulating material coated with an electrically conductive layer.

8. A device as claimed in anyone of the preceding claims, characterized in that the ends of the conductor are connected with the earth terminal (3) by means of electric conductors (23, 24) which may be provided with an insulating sheath (280).

### Patentansprüche

1. Erdungsvorrichtung mit einem langgestreckten elektrischen Leiter (2), der in einer Ebene in einer symmetrischen Form gebogen ist, so daß seine Enden (7) durch einen kleinen Abstand voneinander getrennt sind, dadurch gekennzeichnet, daß die Mitte des Leiters (2) mit einer Erdungsklemme (3) verbunden ist.

2. Erdungsvorrichtung nach Anspruch 1, gekennzeichnet durch, Positioniermittel zum Anordnen der Ebene des Leiters in einer im wesentlichen horizontalen Stellung.

3. Erdungsvorrichtung nach Anspruch 2, dadurch gekennzeichnet, daß die Positioniermittel in Form eines Bodenstiftes (4) gestaltet sind, der an der Erdungsklemme (3) befestigt ist und in einer im wesentlichen senkrechten Stellung zu der Ebene des Leiters in den Boden gesteckt werden kann.

4. Erdungsvorrichtung nach Anspruch 1 für das Tragen am Körper, gekennzeichnet durch eine elektrisch leitende Tragekette (14), die mit der Erdungsklemme verbunden ist.

5. Erdungsvorrichtung nach einem der vorhergehenden Ansprüche, dadurch gekennzeichnet, daß die Oberflächen der Enden des Leiters flach sind und genau mit der Symmetrieebene des Leiters übereinstimmten.

6. Erdungsvorrichtung nach einem der vorhergehenden Ansprüche, dadurch gekennzeichnet, daß die Fläche, die durch den Leiter (2) umschlossen ist, aufgrund des Radius des gewünschten Wirkungsbereiches ausgewählt ist.

7. Erdungsvorrichtung nach einem der vorhergehenden Ansprüche, dadurch gekennzeichnet, daß der Leiter aus einem Band oder Draht aus elektrisch isolierendem Material, das von einer elektrisch leitenden Schicht ummantelt ist, besteht.

8. Erdungsvorrichtung nach einem der vorhergehenden Ansprüche, dadurch gekennzeichnet, daß die Enden des Leiters mit der Erdungsklemme (3) durch elektrische Leiter (23, 24) verbunden sind, die mit einer isolierenden Hülle (28) versehen werden können.

## Revendications

1. Dispositif de mise à la terre comportant un conducteur électrique allongé (2) cintré en un plan de façon symétrique de telle sorte que ses extrémités (7) soient faiblement écartées, caractérisé en ce que le centre du conducteur (2) est relié à une borne de mise à la terre (3).

2. Dispositif de mise à la terre selon la revendication 1, caractérisé par des moyens de positionnement permettant de disposer le plan du conducteur dans une position sensiblement horizontale.

3. Dispositif de mise à la terre selon la revendication 2, caractérisé en ce que les moyens de positionnement présentent la forme d'un axe conducteur (4) fixé à la borne de mise à la terre (3) et prévu pour être mis à la terre dans une position sensiblement perpendiculaire au plan du conducteur.

4. Dispositif de mise à la terre selon la revendication 1, prévu pour être porté sur le corps, caractérisé par une chaîne support électriquement conductrice (14) reliée à la borne de mise à la terre.

5. Dispositif de mise à la terre selon l'une des revendications précédentes, caractérisé en ce que les surfaces des extrémités du conducteur sont plates et coïncident sensiblement avec le plan de symétrie du conducteur.

6. Dispositif de mise à la terre selon l'une quelconque des revendications précédentes, caractérisé en ce que la surface entourée par le conducteur (2) est choisie en se basant sur le rayon de la zone active désirée.

7. Dispositif de mise à la terre selon l'une quelconque des revendications précédentes, caractérisé en ce que le conducteur est une bande ou un fil en matériau électriquement isolant revêtu d'une couche électriquement conductrice.

8. Dispositif de mise à la terre selon l'une quelconque des revendications précédentes, caractérisé en ce que les extrémités du conducteur sont reliées à la borne de mise à la terre (3) par l'intermédiaire de conducteurs électriques (23, 24) qui peuvent être équipés d'une gaine isolante (28).

FIG. 2

FIG.1

FIG.3

FIG.4

FIG.5

FIG.6

FIG. 7

0 098 656

FIG.8

FIG.9

FIG.10

2